(19) Europäisches Patentamt
European Patent Office
Office européen des brevets

(11) **EP 3 643 310 B1**

(12) **EUROPEAN PATENT SPECIFICATION**

(45) Date of publication and mention
of the grant of the patent:
**24.11.2021 Bulletin 2021/47**

(21) Application number: **19204217.4**

(22) Date of filing: **21.10.2019**

(51) Int Cl.:
*A61K 31/415* (2006.01)  *A61K 31/425* (2006.01)
*A61K 31/437* (2006.01)  *A61K 31/445* (2006.01)
*A61P 17/10* (2006.01)  *A61P 19/10* (2006.01)
*A61P 35/00* (2006.01)  *A61P 43/00* (2006.01)
*A61Q 19/08* (2006.01)

(54) **COMPOSITION FOR REVERSE CONTROL OF CELLULAR SENESCENCE COMPRISING PDK1 INHIBITOR**

ZUSAMMENSETZUNG ZUR UMKEHRUNG DER ZELLULÄREN SENESZENZ, PDK1-INHIBITOR UMFASSEND

COMPOSITION DE CONTRÔLE INVERSE DE LA SÉNESCENCE CELLULAIRE COMPRENANT UN INHIBITEUR PDK1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.10.2018 KR 20180128184**

(43) Date of publication of application:
**29.04.2020 Bulletin 2020/18**

(73) Proprietor: **Korea Advanced Institute Of Science And Technology**
**Daejeon, 34141 (KR)**

(72) Inventors:
• **CHO, Kwang Hyun**
  **Daejeon 34141 (KR)**
• **LEE, Soo Beom**
  **Gyeonggi-do 16700 (KR)**
• **AN, Su Gyun**
  **Daejeon 35225 (KR)**
• **KANG, Jun Soo**
  **Seoul 02598 (KR)**
• **CHO, Si Young**
  **Gyeonggi-do 16960 (KR)**
• **KIM, Hyung Su**
  **Gyeonggi-do 17074 (KR)**

(74) Representative: **Cabinet Becker et Associés**
**25, rue Louis le Grand**
**75002 Paris (FR)**

(56) References cited:
**WO-A1-2012/058174    WO-A2-2012/104007**

• **HANNAH E. WALTERS ET AL: "Reversal of phenotypes of cellular senescence by pan-mTOR inhibition", AGING (ALBANY, NY), vol. 8, no. 2, 1 February 2016 (2016-02-01), pages 231-244, XP55658657, US ISSN: 1945-4589, DOI: 10.18632/aging.100872**
• **SHAOBIN YANG ET AL: "Reducing the Levels of Akt Activation by PDK1 Knock-in Mutation Protects Neuronal Cultures against Synthetic Amyloid-Beta Peptides", FRONTIERS IN AGING NEUROSCIENCE, vol. 9, 8 January 2018 (2018-01-08), XP55658715, DOI: 10.3389/fnagi.2017.00435**
• **HANNAH WALTERS ET AL: "mTORC Inhibitors as Broad-Spectrum Therapeutics for Age-Related Diseases", INT. J. MOL. SCI., vol. 19, no. 8, 8 August 2018 (2018-08-08) , page 2325, XP55658674, ISSN: 1661-6596, DOI: 10.3390/ijms19082325**
• **CHUNMEI YANG ET AL: "PDK1 inhibitor GSK2334470 exerts antitumor activity in multiple myeloma and forms a novel multitargeted combination with dual mTORC1/C2 inhibitor PP242", ONCOTARGET, vol. 8, no. 24, 13 June 2017 (2017-06-13), XP55658397, DOI: 10.18632/oncotarget.16642**
• **CHRISTIAN M BEAUSÉJOUR ET AL: "Reversal of human cellular senescence: roles of the p53 and p16 pathways", THE EMBO JOURNAL, vol. 22, no. 16, 1 January 2003 (2003-01-01), pages 4212-4222, XP55658837,**

**(Cont. next page)**

- GURLEEN KAUR ET AL: "Current Progress in the Rejuvenation of Aging Stem/Progenitor Cells for Improving the Therapeutic Effectiveness of Myocardial Repair", STEM CELLS INTERNATIONAL, vol. 2018, 1 January 2018 (2018-01-01), pages 1-9, XP55658654, US ISSN: 1687-966X, DOI: 10.1155/2018/9308301
- CASTEL PAU ET AL: "PDK1-SGK1 Signaling Sustains AKT-Independent mTORC1 Activation and Confers Resistance to PI3K[alpha] Inhibition", CANCER CELL, CELL PRESS, US, vol. 30, no. 2, 21 July 2016 (2016-07-21), pages 229-242, XP029678738, ISSN: 1535-6108, DOI: 10.1016/J.CCELL.2016.06.004
- LEI QI ET AL: "PDK1-mTOR signaling pathway inhibitors reduce cell proliferation in MK2206 resistant neuroblastoma cells", CANCER CELL INTERNATIONAL, vol. 15, no. 1, 29 September 2015 (2015-09-29), XP55658986, DOI: 10.1186/s12935-015-0239-4

**Description**

<u>TECHNICAL FIELD</u>

The present disclosure relates to a reverse-cellular senescence inducing method that uses a specific molecular target to return senescent cells to a young cell state. Specifically, the present disclosure relates to a reverse-cellular senescence inducing composition comprising PDK1 (phosphoinositide-dependent kinase-1) inhibitor or a reverse-cellular senescence inducing method comprising a step of applying the PDK1 inhibitor.

<u>BACKGROUND</u>

Cellular senescence is known as irreversible life phenomenon in which cells damaged by environmental stress permanently stop their cell cycle before they become cancerous. Cellular senescence is known to have a senescence associated secretory phenotype (SASP), along with permanently ceased cell division. Recently, one after the other, it is announced that a substance released from the senescent cells may accelerate senescence of surrounding cells, as well as may cause a number of diseases including a cancer. Thus, a series of attempts to control the senescent cells are being carried out. Most attempts, however, focus on strategies that characteristically kill the senescent cells. To date, it has not been confirmed whether the senescent cells can be reverted back to young cells to prevent the side effects from the senescent cells. The studies indirectly show that the senescent cells may escape cellular senescence via molecular biological manipulations. However, these studies have limitations in making it impossible to fine-tune the cells for reversing the cellular senescence, due to a lack of understanding of the mechanisms of cellular senescence. To date, no molecular target has been suggested that can effectively reverse the senescent cells into normal young cells.
Further, cellular senescence is a phenomenon caused by accumulation of DNA damage inside cells and by various transporters outside the cell. To date, three major signaling pathways leading to cellular senescence are known to be a IGF1-PI3K-mTOR signaling pathway responsible for cell growth and division, a DNA damage-P53-RB signaling pathway, and a NF-kB signaling pathway which regulates SASP. In order to understand the cellular senescence in which various signaling systems are collectively involved, a network based approach is required.

<u>SUMMARY</u>

Thus, the present inventors constructed a cellular senescence network model through a systems biology approach based on an understanding of the cellular senescence and suggested an optimal target for cellular senescence control. More specifically, we integrated three major signaling pathways based on previous literature, and estimated parameters for the network using phosphoprotein array data, and then confirmed that PDK1 is an optimal molecular target for reversing a senescent cell state into a young cell state by using our network model. Thus, we confirmed that inhibition of PDK1 could reverse the senescence of human dermal fibroblasts.
The invention is set out in the appended set of claims.
A purpose of the present disclosure is to provide a reverse-cellular senescence inducing composition comprising a PDK1 (phosphoinositide-dependent kinase-1) inhibitor.
A purpose of the present disclosure is to provide a pharmaceutical composition for prevention or treatment of cellular senescence or age-related diseases, the composition comprising the PDK1 inhibitor.
A purpose of the present disclosure is to provide a reverse-cellular senescence inducing method that involves applying the PDK1 inhibitor *in vitro.*
A purpose of the present disclosure is to provide the PDK1 inhibitor for use in treatment of cellular senescence or age-related diseases.
An exemplary aspect of the present disclosure provides a reverse—cellular senescence inducing composition that contains a PDK1 inhibitor.
Further, another exemplary aspect of the present disclosure provides a pharmaceutical composition for prevention or treatment of cellular senescence or age-related diseases, the composition comprising the PDK1 inhibitor.
Further, another exemplary aspect of the present disclosure provides a health functional food for prevention or amelioration of cellular senescence or age-related diseases, the food comprising the PDK1 inhibitor.
Further, another exemplary aspect of the present disclosure provides a reverse-cellular senescence inducing method that involves applying the PDK1 inhibitor *in vitro.*
Further, another exemplary aspect of the present disclosure provides a method for screening an agent for prevention or treatment of age-related diseases.
Yet another exemplary aspect of the present disclosure provides a PDK1 inhibitor for use in treatment of cellular senescence or age-related diseases.
According to the exemplary aspects of the present disclosure, the PDK1 is a protein that regulates both mTOR and NF-kB, which are key signaling proteins involved in cellular senescence. Thus, the PDK1 is a key protein that determines growth, division and SASP of cells. Temporary inhibition of the PDK1 effectively reversed some of the senescent cells into normal young cells, and the phenotype of the senescent cells was no longer observed in the reversed cells. Thus, PDK1 opens up new possibilities for current aging treatment strategies, which have focused on inducing apoptosis of senescent cells. The PDK1 may be usefully used in development of anti-aging substances and an agent for treating age-related diseases.

## BRIEF DESCRIPTION OF THE DRAWINGS

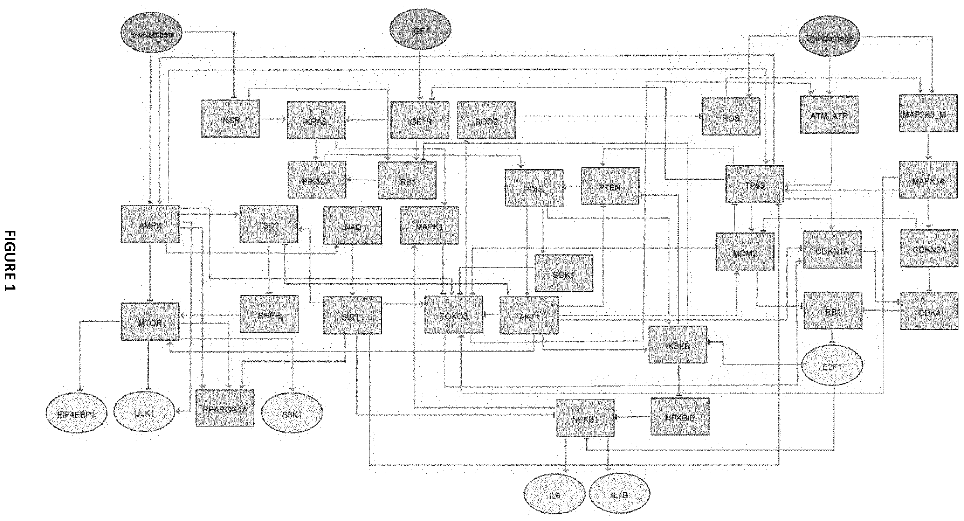

FIG. 1 shows a cellular senescence network model consisting of 41 nodes which is constructed based on previous literature. A green arrow indicates an activation link and a red T-arrow indicates an inhibition link. There are three types of nodes: purple indicates input nodes (3 total), green ellipse indicates output nodes (6 total), and blue square indicates internal nodes (32 total).

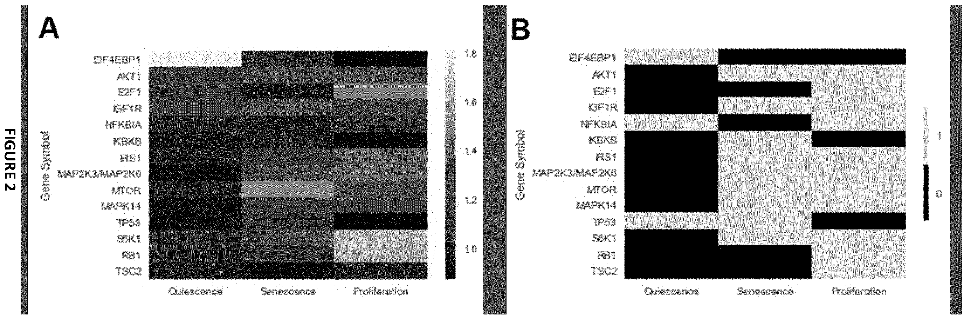

FIG. 2 shows a heat map of a result of the phosphoprotein array on fibroblasts. Each row of the heat map represents a cellular state (a temporarily suspended state: quiescence, a permanently ceased sate: senescence, and a division state: proliferation). Each column thereof represents a gene symbol. FIG. 2A shows an area below a curve of a time-series phosphoprotein array data corresponding to each gene. FIG. 2B shows a result of converting the area under curve value into 0 and 1.

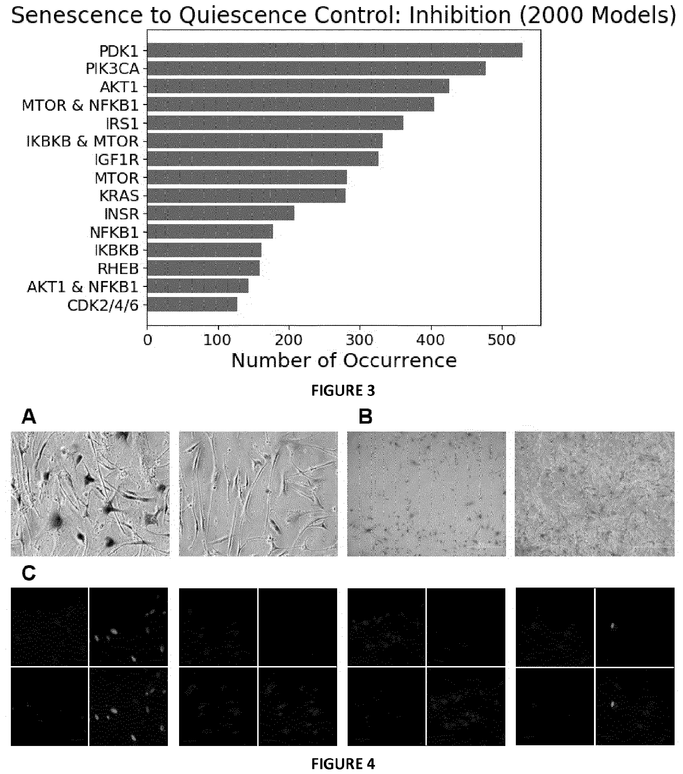

FIG. 3 shows a result of training 2000 network models using an evolutionary algorithm and then applying the trained models to an cellular senescence network model constructed in accordance with the present disclosure for large scale simulation. The y-axis represents a gene symbol of an inhibition target, and the x-axis represents the number of models representing the inhibition simulation result among the 2000 models.

FIG. 4 shows a results of a cell experiment in which a PDK1 inhibitor (BX-795; Selleckchem) is applied to senescent fibroblasts. FIG. 4A shows a result of SA β-gal activity assay. FIG. 4B shows a result of wound healing assay. FIG. 4C shows a result of ki-67 fluorescence staining. In a direction from left to right, normal young cells, senescent cells, senescent cells treated with mTOR inhibitor and senescent cells treated with PDK1 inhibitor are shown.

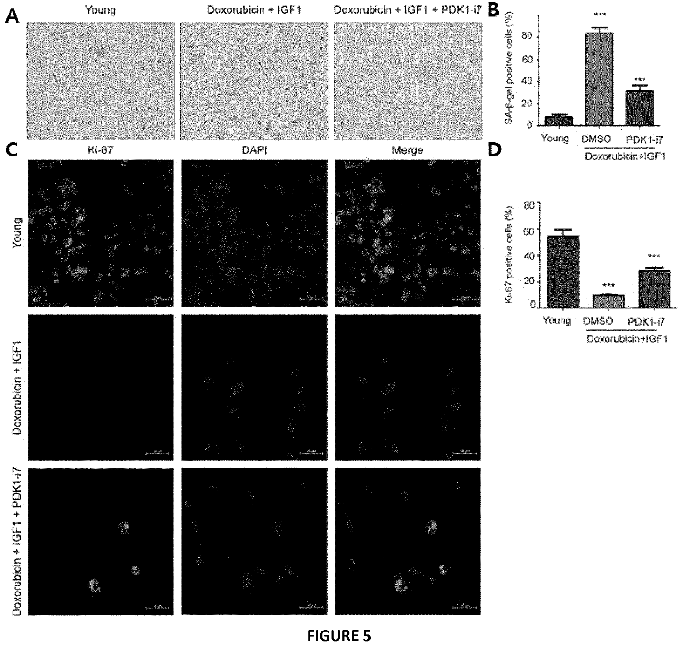

FIG. 5 shows a result of a cell experiment in which PDK1 inhibitor (PDK1 inhibitor 7) is applied to senescent fibroblasts. FIG. 5A, 5B show a result of SA β-gal activity assay. FIG. 5C, 5D show a result of ki-67 fluorescence staining.

## DETAILED DESCRIPTION

In the following detailed description, reference is made to the accompanying drawing, which forms a part hereof.

Hereinafter, the present disclosure will be described in detail.

The present disclosure provides a reverse-cellular senescence inducing composition comprising a PDK1 (phosphoinositide-dependent kinase-1) inhibitor.

As used herein, the term "reverse-cellular senescence (or reverse control)" refers to reverting a senescence state back to a young state by fundamentally controlling the expression of genetic factors that induce cellular senescence, unlike the anti-aging concept which is preventing and treating the aging.

Despite the fact that anti-aging and age-related diseases have been actively researched, current anti-aging and age-related disease treatment strategies focus on killing senescent cells that release harmful chemicals and adversely affect surrounding cells. Therefore, practically all anti-aging therapies fail to demonstrate reliable efficacy and stability in clinical trials. The present disclosures sought to determine whether it is possible to return senescent cells to the quiescence state while excluding random proliferation.

First, in one aspect of the present disclosure, a cellular senescence network model was constructed to identify an optimal molecular target for reversing a senescent cell state to a young cell state. Subsequently, a phosphoprotein array of fibroblasts was performed. Then, we applied the 2000 network models trained through the evolutionary algorithm to the cellular senescence network model according to the present disclosure and then confirmed that the PDK1 is the optimal molecular target. Finally, it was confirmed that when the PDK1 inhibitor is applied to senescent fibroblasts, the senescence state of the cells decreases and the cells grow and divide again when the cells are cultured in a medium under normal conditions. In this way, the present inventors confirmed that the PDK1 inhibitor in accordance with the present disclosure could be usefully used as a reverse-cellular senescence inducing composition.

In the present disclosure, the PDK1 inhibitor may be an expression inhibitor of the PDK1 gene or an activity inhibitor of the PDK1 protein. The expression inhibitor of the PDK1 gene may be at least one selected from a group consisting of antisense oligonucleotides, small interfering RNA (siRNA), small hairpin RNA (shRNA), and miRNA (microRNA) that complementarily bind to mRNA of a PDK1 gene or a gene that promotes PDK1 expression, but may not be limited thereto. Further, the activity inhibitor of the PDK1 protein may be at least one selected from a group consisting of antibodies specifically binding to the PDK1 protein, and antigen-binding fragments, compounds, peptides, peptide mimetics and aptamers thereof, but may not be limited thereto. Preferably, the activity inhibitor of the PDK1 protein may include a compound that directly/indirectly binds to PDK1 protein and inhibits activity thereof. The compound may include, but is not limited to, BX-795, BX-912, PHT-427, GSK2334470, OSU-03012, PDK1 inhibitor 7, and the like. In one example of the present disclosure, the PDK1 inhibitors, BX-795 and PDK1 inhibitor 7 ($C_{28}H_{22}F_2N_4O_4$, CAS No. 1001409-50-2) were used as the compound that inhibits the PDK1 activity.

As used herein, the term "antisense oligonucleotide" refers to DNA or RNA or derivatives thereof containing a nucleic acid sequence complementary to a sequence a specific mRNA. The antisense oligonucleotide binds to a complementary sequence in mRNA and thus acts to inhibit translation of the mRNA into protein. The antisense sequence means a DNA or RNA sequence complementary to the mRNA of the gene and capable of binding to the mRNA. The antisense sequence may suppress essential activity for translation, translocation into cytoplasm, maturation or any other overall biological functions of the mRNA.

Further, the antisense nucleic acid may be modified at one or more bases, sugar or backbone positions thereof to enhance efficacy thereof. The nucleic acid backbone may be modified into phosphorothioate, phosphotriester, methylphosphonate, short chain alkyl, cycloalkyl, short chain heteroatomic, heterocyclic inter-saccharide linkages and the like. Further, the antisense nucleic acid may contain one or more substituted sugar moieties. The antisense nucleic acid may contain a modified base. The modified base may include hypoxanthine, 6-methyladenine, 5-methylpyrimidine (especially 5-methylcytosine), 5-hydroxymethylcytosine (HMC), glycosyl HMC, gentobiosil HMC, 2-aminoadenine, 2-thiouracil, 2-thiothymine, 5-bromouracil, 5-hydroxymethyluracil, 8-azaguanine, 7-deazaguanine, N6(6-aminohexyl)adenine, 2,6-diaminopurine, etc. Further, the antisense nucleic acid may be chemically bound with one or more moieties or conjugates that enhance the activity and cellular adsorption of the antisense nucleic acid. The moieties may include at-soluble moieties such as cholesterol moieties, cholesteryl moieties, cholic acids, thioethers, thiocholesterols, fatty chains, phospholipids, polyamines, polyethylene glycol chains, adamantane acetic acid, palmityl moiety, octadecylamine, hexylaminocarbonyl-oxycholesterol moiety, and the like, but are not limited thereto. The antisense oligonucleotide may be synthesized *in vitro* in a conventional manner and administered *in vivo.* Alternatively, the antisense oligonucleotide may be synthesized *in vivo.*

As used herein, the term "siRNA" refers to a nucleic acid molecule capable of mediating RNA interference or gene silencing. Since siRNAs can inhibit the expression of target genes, they are provided for efficient gene knockdown method or gene therapy methods.

The siRNA molecule in accordance with the present disclosure may have a double chain structure in which a sense strand (a sequence corresponding to the mRNA sequence of the PDK1 gene as a target gene according to an aspect of the present disclosure) and an antisense strand (a sequence complementary to the mRNA sequence) are positioned at opposite sides. The siRNA molecule in accordance with the present disclosure may have a single chain structure with a self-complementary sense and an antisense strand. Furthermore, siRNAs are not limited to the complete paring of double-stranded RNA portions in which RNAs pair with each other. Rather, the unpaired portion due to a mismatch (the corresponding base is not complementary thereto), a bulge (no base corresponding to one of the chains), and the like may be contained in the siRNA. Further, as long as the siRNA terminal structure can inhibit the expression of the target gene by RNAi effect, the siRNA terminal structure may be both blunt terminal and a cohesive terminal. The cohesive terminal structure may include both 3'-terminal protruding structure and 5'-terminal protruding structure. The method for preparing siRNA includes a method of directly synthesizing siRNA *in vitro,* and then introducing siRNA into a cell via a transformation process, and a method of transforming or infecting, into the cells, a siRNA expression vector or a PCR-derived siRNA expression cassette as produced so that a siRNA is expressed in a cell.

As used therein, the term "shRNA" refers to small hairpin RNA or short hairpin RNA and is used to silence genes via RNA interference. Usually, the shRNA may be introduced into the target cell using a vector. The shRNA hairpin structure may be cleaved by other substances in the cell to become siRNA.

As used herein, the term "antibody" refers to a substance that reacts when an external substance, an antigen invades while the antibody is circulating in blood or lymph in an immune system of a living body. The antibody is globulin-based protein formed in a lymphoid tissue, also known as immunoglobulins. The antibody is a protein produced by B cells and flowing into body fluids and binds specifically to an antigen. One antibody molecule has two heavy chains and two light chains. Each of the heavy and light chains has a variable region at a N-terminal thereof. Each variable region is composed of three complementarity determining regions (CDRs) and four framework regions (FRs). The CDRs determine the antigen-binding specificity of the antibody and is present in a form of a relatively short peptide sequence that is maintained by the FRs in the variable region. For the purpose of the present disclosure, the antibody may be an antibody capable of binding to the PDK1 and inhibiting the activity of the PDK1.

Further, in accordance with the present disclosure, the PDK1 inhibitor may be characterized by inhibiting the transcriptional activity of the mammalian target of rapamycin (mTOR) and nuclear factor kappa B (NF-kB). The PDK1 inhibitor according to the present disclosure may therefore be characterized by simultaneous regulation of mTOR and NF-kB.

The reverse-cellular senescence inducing composition according to the present disclosure may be applied both *in vivo* and *in vitro.* For example, the reverse—cellular senescence inducing composition according to the present disclosure may be used in various forms such as a pharmaceutical composition, a nutraceutical composition, a cosmetic composition, a composition as added to a medium, and a reagent composition.

Further, the present disclosure provides a pharmaceutical composition for the prevention or treatment of cellular senescence or age-related diseases, the composition comprising the PDK1 inhibitor.

As used herein, the term "Senescence(or aging)" refers to all physiological changes in the body that occur over time, and refers to life phenomena that are caused by a variety of factors, depending on the individual. The term "Cellular senescence" refers to senescence in cell level driven by a variety of stresses, including DNA damage. Specifically, aging occurs in each organ and tissue, which results from the senescence of the cells (cellular senescence) that constitute the organism.

In accordance with the present disclosure, the age-related disease may include at least one selected from a group consisting of hair loss, wrinkle, humpback, osteoporosis, rheumatoid arthritis, asthma, dermititis, psoriasis, cystic fibrosis, post transplantation late and chronic solid organ rejection, multiple sclerosis, systemic lupus erythematosus, Sjogren's syndrome, Hashimoto thyroiditis, polymyositis, scleroderma, Addison's disease, vitiligo, pernicious anemia, glomerulonephritis, pulmonary fibrosis, inflammatory bowel disease, autoimmune diabetes, diabetic retinopathy, rhinitis, ischemia-reperfusion injury, post-angioplasty restenosis, chronic obstructive pulmonary diseases (COPD), Grave's disease, gastrointestinal allergies, conjunctivitis, atherosclerosis, coronary artery disease, angina, cancer, arteriole disease, and graft-versus-host disease. Preferably, the age-related disease may be at least one selected from a group consisting of rheumatoid arthritis, dermititis, cystic fibrosis, pulmonary fibrosis, inflammatory bowel disease, atherosclerosis, coronary artery disease, and cancer, but is not limited thereto.

In accordance with the present disclosure, the pharmaceutical composition may be in a form of capsules, tablets, granules, injectables, ointments, powders or beverages. The pharmaceutical composition may target humans. The pharmaceutical composition may be prepared into an oral dosage form such as powders, granules, capsules, tablets, and aqueous suspensions, or into external application preparations, suppositories, and sterile injectable solutions according to conventional methods, but may not be limited thereto.

The pharmaceutical composition according to the present disclosure may contain a pharmaceutically acceptable carrier. In oral administration of the composition, the pharmaceutically acceptable carrier may include binders, lubricants, disintegrants, excipients, solubilizers, dispersants, stabilizers, suspending agents, pigments, fragrances, etc. In injection of the composition, the pharmaceutically acceptable carrier may include buffers, preservatives, analgesics, solubilizers, isotonic agents, stabilizers, etc. which may be mixed with the composition. For topical administration thereof, the pharmaceutically acceptable carrier may include bases, excipients, lubricants, preservatives and the like. The formulation of the pharmaceutical composition according to the present disclosure may be prepared in various ways by mixing the composition with the pharmaceutically acceptable carrier as described above. For example, in the oral administration thereof, the formulation of the pharmaceutical composition may be in the form of tablets, troches, capsules, elixirs, suspensions, syrups, wafers, etc. As an injectable, the formulation of the pharmaceutical composition may be prepared in a unit dose ampoule or in multiple dose forms.

For example, the suitable carriers, excipients and diluents suitable for the formulation may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate or mineral oil and the like. Further, the composition may contain additional fillers, anticoagulants, lubricants, wetting agents, fragrances, emulsifiers, and preservatives.

A route of administration of the pharmaceutical composition according to the present disclosure may include oral, intravenous, intramuscular, intraarterial, intramedullary, intradural, intracardiac, transdermal, subcutaneous, intraperitoneal, intranasal, intestinal, topical, sublingual or rectal, but may not be limited thereto. However, the route of administration of the pharmaceutical composition according to the present disclosure is not limited thereto. The "parenteral" may include subcutaneous, intradermal, intravenous, intramuscular, intraarticular, intrabursal, intrasternal, intradural, intralesional and intracranial injection or infusion techniques. The pharmaceutical composition according to the present disclosure may be administered in the form of suppositories for rectal administration.

The pharmaceutical composition according to the present disclosure may vary depending on many factors including activity of a specific effective component, age, weight, general health, sex, formulation, time of administration, route of administration, rate of release, drug combination and severity of a specific disease to be prevented or treated. The dosage of the pharmaceutical composition may vary depending on the patient's condition, weight, extent of disease, drug form, route of administration and duration, but may be appropriately selected by those skilled in the art. Preferably, while taking into account all of the factors, the composition may be administered in an amount that can achieve the maximum effect in a minimum amount without side effects. More preferably, the composition may be repeatedly administered several times a day at an effective dose of 1 to 10000 μg/kg body weight/day, even more preferably, 10 to 1000 mg/kg body weight/day. The dosage does not limit the scope of the present disclosure in any way.

Cellular senescence according to the present disclosure may include senescence of at least one selected from a group consisting of fibroblast, vascular endothelial cells, myofibroblasts, osteoblasts, chondrocytes, cardiomyocytes, hematopoietic stem cells, hepatocytes, pancreatic cells, germ cells and keratinocytes, preferably, fibroblast or vascular endothelial cells, but may not be limited thereto.

The pharmaceutical composition for the prevention or treatment of the cellular senescence or age-related diseases according to the present disclosure may additionally be co-administered with other agents for prevention or treatment of age-related diseases. Further, the pharmaceutical composition for the prevention or treatment of the cellular senescence or age-related diseases according to the present disclosure may be used alone or in combination with surgery, radiation therapy, hormone therapy, chemotherapy and biological response modifiers.

Further, the present disclosure provides a health functional food for the prevention or improvement of cellular senescence or age-related diseases, the food comprising a PDK1 inhibitor.

The health functional food according to the present disclosure can be manufactured and processed in the form of tablets, capsules, powders, granules, liquids, and pills, etc for prevention and improvement of cellular senescence or age-related diseases.

As used herein, the term "health functional food" refers to foods manufactured and processed using raw materials or ingredients with useful functions for human body in accordance with the Law No. 6727 on Health Functional Foods The health functional food means the food as ingested for the purpose of regulating nutrients related to the structure and function of the human body or obtaining a beneficial effect for health uses such as physiological action.

The health functional food according to the present disclosure may contain common food additives. The qualification of the food additives may comply with the provisions of the Food and Drug Administration Regulations as approved by the FDA and with General Regulations for Food Additives and General Testing Methods as approved by the FDA unless otherwise defined.

Commercially available items listed in the food additive code include, for example, chemical compounds such as ketones, glycine, calcium citrate, nicotinic acid, and cinnamic acid. The items included in the food additive code include natural additives such as persimmon pigment, licorice extract, crystalline cellulose, high pigment, and guar gum, and mixed preparations such as a sodium L-glutamate preparation, a noodle-added alkaline agent, a preservative agent, and a tar coloring agent.

For example, a health functional food in a tablet form may be prepared by mixing an inhibitor of PDK1 as an active ingredient in accordance with the present disclosure with an excipient, a binder, a disintegrant and other additives, and then granulating the mixture and then adding the lubricant thereto and compression-molding the mixture, or directly compression-molding the mixture. Further, the food functional food in a tablet form may contain bitters if necessary.

A health functional food in a form of a hard capsule among capsules may be prepared by filling a mixture of additives such as excipients with an inhibitor of PDK1 as an active ingredient in accordance with the present disclosure into a conventional hard capsule. A health functional food in a form of a soft capsule may be prepared by filling a capsule base such as gelatin with a mixture of an inhibitor of PDK1 as an active ingredient with additives such as excipients. The soft capsule may contain a plasticizer such as glycerin or sorbitol, a colorant, a preservative, and the like, as necessary.

A health functional food in a form of a pill may be prepared by molding a mixture of an inhibitor of PDK1 as an active ingredient in accordance with the present disclosure, an excipient, a binder, a disintegrant, and the like using a known method. The pill may be coated with sugar or other coatings as needed. Alternatively, the surface of the fill may be coated with a material such as starch or talc.

The health functional food in a form of granule may be prepared by granulating a mixture of an inhibitor of PDK1 as an active ingredient in accordance with the present disclosure, an excipient, a binder, a disintegrant, etc. in a conventional method. If necessary, the granule may contain a flavoring agent, bitters and the like.

The health functional food may include beverages, meat, chocolate, foods, confectionery, pizza, ramen, other noodles, gums, candy, ice cream, alcoholic beverages, vitamin complexes and dietary supplements.

Further, the present disclosure provides a reverse-cellular senescence inducing method that involves applying the PDK1 inhibitor to senescent cells *in vitro.*

Further, the present disclosure provides a method of screening an agent for prevention or treatment of age-related diseases, the method comprising (a) applying a candidate substance to an isolated senescent cell; (b) measuring a PDK1 expression or activity level in the senescent cell to which the candidate substance has been applied; (c) when the PDK1 expression or activity level measured in the step (b) is lower than a PDK1 expression or activity level measured in an isolated senescent cell to which the candidate substance is not applied, determining that the candidate substance can be used as an agent for prevention or treatment of age-related diseases.

According to the screening method in accordance with the present disclosure, first, a candidate substance to be analyzed may first contact an isolated senescent cells. The candidate substances are unknown substances used in the screening to test whether the substances affect the expression level, protein amount, or protein activity of PDK1. The substance may include, but is not limited to, a chemical, antisense oligonucleotide, antisense-RNA, siRNA, shRNA, miRNA, an antibody specific to the protein or natural extract.

Then, the expression level of the gene, the amount of protein or activity of the protein in the cell to which the candidate substance is applied may be measured. When a decrease in the expression level of the gene, the amount of the protein or the activity of the protein is measured, the candidate substance could be determined to be used as an agent to treat or prevent age-related diseases.

In accordance with the present disclosure, a method for measuring the expression level of the gene or the amount of the protein may include a known process for separating mRNA or protein from a biological sample using known techniques. As used herein, the term "biological sample" refers to a sample taken from a living body in which the expression level of the gene or the protein level is different from that of a normal control group. The sample may include, but is not limited to, tissue, cells, blood, serum, plasma, saliva, and urine.

The measurement of the expression level of the gene may be preferably the measurement of the level of mRNA. The method of measuring the mRNA level may include, but not be limited to, reverse transcriptase polymerase chain reaction (RT-PCR), real time reverse transcriptase chain reaction, RNase protection assay, Northern blot and DNA chip.

The measurement of the protein level may use an antibody. In this case, a marker protein in the biological sample and the antibody specific thereto form a conjugate, an antigen-antibody complex. The amount of the antigen-antibody complex as formed may be measured quantitatively based on a magnitude of a signal of a detection label. Such a detection label may be selected from a group consisting of enzymes, fluorescent materials, ligands, luminescent materials, microparticles, redox molecules and radioisotopes. However, the present disclosure is not limited thereto. An analytical method for measuring the protein level may include western blotting, ELISA (enzyme linked immunosorbent assay), RIA (radioimmunoassay), radial immunodiffusion, Ouchterlony immunodiffusion, rocket immune electrophoresis, immune histochemical staining, immunoprecipitation assay, complement fixation assay, immunofluorescence, immunochromatography, FACS (fluorescence activated cell sorter analysis) or protein chip technology, but may not be limited thereto.

Further, the present disclosure provides a PDK1 inhibitor for use in treatment of cellular senescence or age-related diseases.

Terms not otherwise defined herein have a meaning commonly used in the art to which the present disclosure belongs.

Hereinafter, the present disclosure will be described in detail by examples.

However, the following examples are merely illustrative of the present disclosure and the contents of the present disclosure are not limited to the following examples.

**Example 1:** Cellular Senescence **Network Model Construction**

An cellular senescence network model composed of 41 nodes was constructed based on previous literature and is shown in FIG. 1.

The network was constructed based on following three major signaling pathways leading to cellular senescence: a IGF1-PI3K-mTOR signaling pathway responsible for cell growth and division; a DNA damage-P53-RB signaling pathway, and a NF-kB signaling pathway responsible for SASP. The previous literature information of 77 links contained in the network is shown in Table 1 below.

[Table 1]

| From | Regulation type | To | Mechanism | PMID |
|---|---|---|---|---|
| AKT1 | - | CDKN1A | phosphorylation | 11463845 |
| AKT1 | - | FOXO3 | phosphorylation | 12517744 |
| AKT1 | + | IKBKB | phosphorylation | 11259436 |
| AKT1 | + | MDM2 | phosphorylation | 11923280 |
| AKT1 | + | MTOR | phosphorylation | 16027121 |
| AKT1 | - | PTEN | ubiquitination | 26183061 |
| AKT1 | - | TSC2 | phosphorylation | 12172553 |
| AMPK | + | FOXO3 | phosphorylation | 22186033 |
| AMPK | - | MTOR | phosphorylation | 22186033 |
| AMPK | + | NAD | metabolism | 19262508 |

8

(continued)

| From | Regulation type | To | Mechanism | PMID |
|---|---|---|---|---|
| AMPK | + | PPARGC1A | transcription | 23963743 |
| AMPK | + | TP53 | phosphorylation | 22186033 |
| AMPK | + | TSC2 | phosphorylation | 22186033 |
| AMPK | + | ULK1 | phosphorylation | 22186033 |
| ATM/ATR | + | TP53 | phosphorylation | 9925639 |
| CDK2/4/6 | - | RB1 | phosphorylation | 24089445 |
| CDKN1A | - | CDK2/4/6 | binding | 24089445 |
| CDKN2A | - | CDK2/4/6 | binding | 24089445 |
| CDKN2A | - | MDM2 | binding | 9724636 |
| DNAdamage | + | ATM/ATR | | |
| DNAdamage | + | MAP2K3/MAP2K6 | | |
| DNAdamage | + | ROS | | |
| FOXO3 | + | ATM/ATR | phosphorylation | 22893124 |
| FOXO3 | + | CDKN1A | transcription | 28808415 |
| FOXO3 | + | SOD2 | transcription | 26184557 |
| IGF1 | + | IGF1R | | |
| IGF1R | + | IRS1 | phosphorylation | 26474286 |
| IGF1R | + | KRAS | GTP-GDP conversion | 23658296 |
| IKBKB | - | IRS1 | phosphorylation | 22982470 |
| IKBKB | - | NFKBIE | phosphorylation | 10602459 |
| IKBKB | - | PTEN | transcription | 14729949 |
| INSR | + | IRS1 | binding | 20966354 |
| INSR | + | KRAS | GTP-GDP conversion | 20966354 |
| IRS1 | + | PIK3CA | binding | 20966354 |
| KRAS | + | MAPK1 | phosphorylation | 11942415 |
| KRAS | + | PIK3CA | binding | 21779497 |
| lowNutrition | + | AMPK | | |
| lowNutrition | - | INSR | | |
| MAP2K3/MAP2K6 | + | MAPK14 | phosphorylation | 11942415 |
| MAPK1 | - | FOXO3 | phosphorylation | 18204439 |
| MAPK14 | + | CDKN2A | transcription | 19619499 |
| MAPK14 | + | FOXO3 | phosphorylation | 22128155 |
| MAPK14 | + | TP53 | phosphorylation | 17481747 |
| MDM2 | - | FOXO3 | ubiquitination | 21238503 |
| MDM2 | - | RB1 | ubiquitination | 15577944 |
| MDM2 | - | TP53 | ubiquitination | 23885265 |
| MTOR | - | EIF4EBP1 | phosphorylation | 12080086 |
| MTOR | + | PPARGC1A | transcription | 18046414 |

(continued)

| From | Regulation type | To | Mechanism | PMID |
|------|-----------------|-----|-----------|------|
| MTOR | + | S6K1 | phosphorylation | 12080086 |
| MTOR | - | ULK1 | phosphorylation | 21258367 |
| NAD+ | + | SIRT1 | chemical reaction | 22106091 |
| NFKB1 | + | IL1B | transcription | 22182507 |
| NFKB1 | + | IL6 | transcription | 22182507 |
| NFKB1 | + | MAPK1 | indirect | 16644866 |
| NFKBIE | - | NFKB1 | binding | 10602459 |
| PDK1 | + | AKT1 | phosphorylation | 25295225 |
| PDK1 | + | IKBKB | indirect | 15802604 |
| PDK1 | + | SGK1 | phosphorylation | 28236975 |
| PIK3CA | + | PDK1 | binding | 25295225 |
| PTEN | - | PDK1 | indirect | 25295225 |
| RB1 | - | E2F1 | transcription | 24089445 |
| RHEB | + | MTOR | binding | 15854902 |
| ROS | + | MAP2K3/MAP2K6 | indirect | 20112989 |
| SGK1 | - | FOXO3 | phosphorylation | 11154281 |
| SIRT1 | + | FOXO3 | deacetylation | 14976264 |
| SIRT1 | - | NFKB1 | deacetylation | 23029496 |
| SIRT1 | + | PPARGC1A | deacetylation | 21396404 |
| SIRT1 | - | TP53 | deacetylation | 12220851 |
| SIRT1 | + | TSC2 | deacetylation | 20169165 |
| SOD2 | - | ROS | chemical reaction | 22302046 |
| TP53 | + | CDKN1A | transcription | 24089445 |
| TP53 | - | IGF1R | indirect | 29273484 |
| TP53 | + | MDM2 | transcription | 14707283 |
| TP53 | + | PTEN | transcription | 11545734 |
| TSC2 | - | RHEB | binding | 12842888 |
| E2F1 | - | IKBKB | binding | 27185527 |
| TP53 | + | AMPK | indirect | 27454290 |

## Example 2: Cell Cycle State Analysis Through Phosphoprotein Array

A phosphoprotein array experiment(reverse phase protein assay, RPPA) was performed on fibroblasts and the results were plotted on a heat map. Specifically, FIG. 2A shows an area below a curve of time-series phosphoprotein array data corresponding to each gene based on a gene symbol and cellular state. The cellular state was divided into Quiescence, Senescence, or Proliferation. FIG. 2B shows a result of converting the area under curve value into 0 and 1, using a weighted sum Boolean logic model. The model refers to a model in which each link has a real number and each node has a base activity so that the node state is set to 0 or 1. For example, in a following drawing below, a state of $x_4$ is 1 when $w_1 * x_1 - w_2 * x_2 + w_3 x_3 + b_{x4}$ is greater than 0, while a state of $x_4$ is 0 when $w_1 * x_1 - w_2 * x_2 + w_3 x_3 + b_{x4}$ is smaller than or equal to 0.

Therefore, the state (0 or 1) of the node contained in the network changes according to the state (0 or 1) of other nodes. If the states of all nodes when the states of all nodes of the network are updated N times using the above method are the same as those of all nodes when the states of all nodes of the network are updated (N+1) times using the above method, the network reaches a fixed attractor. If the states of all nodes when the states of all nodes of the network are updated N times using the above method are the same as those of all nodes when the states of all nodes of the network are updated (N-M) times using the above method, the network reaches a cyclic attractor of a size M (where 1 < M <N).

**Example 3: Optimal Molecular Target Discovery using Cellular Senescence Network Model Simulation**

We trained 2000 network models through evolutionary algorithms. The trained models were applied to the cellular senescence network model constructed in the Example 1 for a large-scale simulation. Thus, an optimal molecular target capable of reversing the senescent cell state into a young cell state was discovered. The evolutionary algorithm is an optimization technique developed to mimic the evolution of living organisms and is suitable for solving discontinuity such as Boolean fitting. In this example, a following cost function below was used.

$$cost = \sum_{i=1}^{n}\sum_{j=1}^{m}(H(x_{ij}) - y_{ij})^2$$

In the function, i means each cellular state (a temporarily suspended state: quiescence, a permanently ceased(stopped) sate: senescence, and a division state: proliferation), and j means each node number of 41 nodes. $x_{ij}$ means a value (0 or 1) of a j th node in a i th state. $H(x_{ij})$ means the value (0 or 1) of the j th node in the i th state in the attractor computed from those inputs. $y_{ij}$ means the value (0 or 1) of the j th node in the i th state from a target attractor, meaning the converted RPPA data value. As a result, the network is trained such that the attractor obtained by applying the input node combination corresponding to the three states of Table 2 below is identical to the converted RPPA data value.

[Table 2]

| Input node | DNAdamage | lowNutrition | IGF1 |
|---|---|---|---|
| Proliferation | 0 | 0 | 1 |
| Quiscence | 1 | 1 | 0 |
| Senescence | 1 | 0 | 1 |

For the 2000 model networks trained through the evolutionary algorithm, the combination of input nodes is set to the permanently ceased state (senescence). Thus, the senescene attractor state is obtained. Then, while the input node combination is maintained, we performed a single inhibition simulation of all nodes and a inhibition simulation of the two nodes combinations. The results are shown in FIG. 3. As shown in FIG. 3, it was confirmed that the PDK1 may be an optimal molecular target for reversing the senescent cell state to a young cell state among gene symbols of inhibition targets.

**Example 4: Checking of Cell Cycle Change in Senescent Cells based on PDK1 Inhibition**

To determine whether the senescent cells subjected to the PDK1 inhibition are reverted to normal young cells, the PDX1 inhibitor BX-795 (Selleckchem) was applied to the senescent fibroblasts. Then, SA β-gal activity assay (senescence-associated β-galactosidase kit), wound healing assay, and fluorescence staining experiments were performed. Thus, the cell cycle change in the senescent cells based on PDK1 Inhibition was checked.

Specifically, cellular senescence was caused by exposing the cells for 7 days to culture conditions causing senescence, containing 100 ng/ml of IGF1, 100 ng/ml of doxorubicin, 10 % FBS, and 4.5 g/L of glucose. At this time, most of the cells turned senescent. Then, the PDK1 inhibitor was applied to the same culture condition for 7 days. Total 14 days of the application lapsed. Then, the cells were treated with growth media free of IGF1, doxorubicin, or PDK1 inhibitor. One day later, SA β-gal activity assay was performed and the results are shown in FIG. 4A. The wound healing assay was performed under the same conditions. The cells were treated with the culture medium of the growth condition for 7 days, and the results are shown in FIG. 4B. Ki-67 expression was analyzed to evaluate the division activity of the cells and the results are shown in 4C.

As shown in FIG. 4A, when the BX-795 was applied to senescent fibroblasts, SA β-gal as an indicator of senescent cells was significantly reduced. Furthermore, it was confirmed from FIG. 4B that when the BX-795 was applied to the senescent fibroblast, the cell rejuvenation ability of the senescent cells was normalized. Further, it was confirmed from FIG. 4C through the ki-67 staining that cell division occurred in some of the cells when the cells were treated with the growth medium at 7 days after the drug application. This confirms that the cells grow and divide again when the cells were treated with the medium of the normal conditions.

In order to check whether the same result occurs when other PDK1 inhibitors are used, SA β-gal activity assay was performed in the same manner using PDK1 inhibitor 7. Senescent cells express SA β-gal and show a blue color. Therefore, the number of bluestained senescent cells as SA β-gal activity assay-positive and the total number of cells were counted using an optical microscope. Then, the percentage of the senescent cells was indicated. In addition, fluorescence staining experiments were performed to check the expression of the cell proliferation marker ki-67. The results are shown in FIG. 5.

As shown in FIG. 5A, 5B, when the PDK1 inhibitor 7 was applied to senescent fibroblasts, the stained cells showed statistically significant reduction by about 40% or greater compared with the PDK1 inhibitor non-treated group. Thus, it was confirmed that the senescence state of cell was significantly reduced. Further, as shown in in FIG. 5C, 5D, the results of fluorescence staining by ki-67 staining showed that the application of the PDK1 inhibitor 7 to the senescent cells significantly increased the number of ki-67 positive cells by 20% or greater, thereby confirming the re-growth and re-division of the cells. This indicates that the reverse-cellular senescence was effectively induced in the senescent cells.

It is conformed from the results that the PDK1 according to the present disclosure is a protein that simultaneously regulates mTOR and NF-KB among the key proteins related to the cellular senescence, and is a key protein that determines growth, division and SASP of cells. Inhibition of the PDK1 effectively reverses some of the senescent cells into normal young cells, and it was confirmed that the phenotype of the senescent cells was no longer observed in the reversed cells. Thus, PDK1 opens up new possibilities for current aging treatment strategies, which have focused on inducing apoptosis of senescent cells. The PDK1 may be usefully used in development of anti-aging substances and an agent for treating age-related diseases.

**Claims**

1.   Use of a composition comprising a PDK1 (phosphoinositide-dependent kinase-1) inhibitor for in vitro reversing a senescent cell, wherein the PDK1 inhibitor is an expression inhibitor of a PDK1 gene or an activity inhibitor of a PDK1 protein.

2.   The use of claim 1, wherein the expression inhibitor of the PDK1 gene is selected from a group consisting of antisense oligonucleotides, small interfering RNA (siRNA), small hairpin RNA (shRNA), and miRNA (microRNA) binding complementarily to mRNA of a gene for promoting PDK1 expression or PDK1 gene.

3.   The use of claim 1, wherein the activity inhibitor of the PDK1 protein is selected from a group consisting of an antibody, and an antigen-binding fragment thereof, a compound, a peptide, a peptide mimetic and an aptamer specifically binding to the PDK1 protein.

4.   The use of claim 1, wherein the PDK1 inhibitor inhibits transcriptional activity of a mammalian target of rapamycin (mTOR) and nuclear factor kappa B (NF-kB).

**5.** The use of claim 1, wherein the senescent cell is a senescent fibroblast or senescent vascular endothelial cells.

**6.** A method for in vitro reversing a senescent cell, comprising applying a PDK1 inhibitor to a senescent cell *in vitro,* wherein the PDK1 inhibitor is an expression inhibitor of a PDK1 gene or an activity inhibitor of a PDK1 protein.

**7.** The method of claim 6, wherein the expression inhibitor of the PDK1 gene is selected from a group consisting of antisense oligonucleotides, small interfering RNA (siRNA), small hairpin RNA (shRNA), and miRNA (microRNA) binding complementarily to mRNA of a gene for promoting PDK1 expression or PDK1 gene.

**8.** The method of claim 6, wherein the activity inhibitor of the PDK1 protein is selected from a group consisting of an antibody, and an antigen-binding fragment thereof, a compound, a peptide, a peptide mimetic and an aptamer specifically binding to the PDK1 protein.

**9.** The method of claim 6, wherein the senescent cell is a senescent fibroblast or senescent vascular endothelial cells.

**Patentansprüche**

**1.** Verwendung einer Zusammensetzung, die einen PDK1-(phosphoinositide-dependent kinase-1; Phosphoinositid-abhängige Kinase-1)-Inhibitor enthält, zur In-vitro-Reversion einer seneszenten Zelle, wobei der PDK1-Inhibitor ein Expressionsinhibitor eines PDK1-Gens oder ein Aktivitätsinhibitor eines PDK1-Proteins ist.

**2.** Verwendung nach Anspruch 1, wobei der Expressionsinhibitor des PDK1-Gens ausgewählt ist aus einer Gruppe bestehend aus Antisense-Oligonukleotiden, kleiner interferierender RNA (siRNA), kleiner Haarnadel-RNA (shRNA) und miRNA (microRNA), die komplementär an mRNA eines Gens zur Förderung der PDK1-Expression oder PDK1-Gens binden.

**3.** Verwendung nach Anspruch 1, wobei der Aktivitätsinhibitor des PDK1 -Proteins ausgewählt ist aus einer Gruppe bestehend aus einem Antikörper und einem Antigen-bindenden Fragment davon, einer Verbindung, einem Peptid, einem Peptidmimetikum und einem Aptamer, spezifisch an das PDK1-Protein bindend.

**4.** Verwendung nach Anspruch 1, wobei der PDK1-Inhibitor die Transkriptionsaktivität eines Säugetier-Ziels von Rapamycin (mTOR) und Nuklearfaktor kappa B (NF-κB) hemmt.

**5.** Verwendung nach Anspruch 1, wobei die seneszente Zelle ein seneszenter Fibroblast oder seneszente vaskuläre Endothelzellen ist/sind.

**6.** Verfahren zur In-vitro-Reversion einer seneszenten Zelle, umfassend das Applizieren eines PDK1-Inhibitors zu einer seneszente Zelle *in-vitro*, wobei der PDK1-Inhibitor ein Expressionsinhibitor eines PDK1-Gens oder ein Aktivitätsinhibitor eines PDK1-Proteins ist.

**7.** Verfahren nach Anspruch 6, wobei der Expressionsinhibitor des PDK1-Gens ausgewählt ist aus einer Gruppe bestehend aus Antisense-Oligonukleotiden, kleiner interferierender RNA (siRNA), kleiner Haarnadel-RNA (shRNA) und miRNA (microRNA), die komplementär an mRNA eines Gens zur Förderung der PDK1-Expression oder PDK1-Gens binden.

**8.** Verfahren nach Anspruch 6, wobei der Aktivitätsinhibitor des PDK1-Proteins ausgewählt ist aus einer Gruppe bestehend aus einem Antikörper und einem Antigen-bindenden Fragment davon, einer Verbindung, einem Peptid, einem Peptidmimetikum und einem Aptamer, spezifisch an das PDK1-Protein bindend

**9.** Verfahren nach Anspruch 6, wobei die seneszente Zelle ein seneszenter Fibroblast oder seneszente vaskuläre Endothelzellen ist/sind.

**Revendications**

**1.** Utilisation d'une composition comprenant un inhibiteur de PDK1 (kinase—1 dépendante du phosphoinositide) pour la réversion in vitro d'une cellule sénescente, dans laquelle l'inhibiteur de PDK1 est un inhibiteur de l'expression d'un gène de PDK1 ou un inhibiteur de l'activité d'une protéine de PDK1.

2. Utilisation selon la revendication 1, dans laquelle l'inhibiteur de l'expression du gène de PDK1 est choisi dans le groupe constitué par des oligonucléotides antisens, des petits ARN interférents (ARNsi), des petits ARN en épingle à cheveux (ARNsh), et des ARNmi (microARN) se liant de manière complémentaire à l'ARNm d'un gène pour promouvoir l'expression de PDK1 ou un gène de PDK1.

3. Utilisation selon la revendication 1, dans laquelle l'inhibiteur de l'activité de la protéine de PDK1 est choisi dans le groupe constitué par un anticorps, un fragment de celui-ci se liant à un antigène, un composé, un peptide, un peptidomimétique et un aptamère se liant spécifiquement à la protéine de PDK1.

4. Utilisation selon la revendication 1, dans laquelle l'inhibiteur de PDK1 inhibe l'activité de transcription d'une cible mammifère de rapamycine (mTOR) et du facteur nucléaire kappa B (NF—κB).

5. Utilisation selon la revendication 1, dans laquelle la cellule sénescente est un fibroblaste sénescent ou une cellule endothéliale vasculaire sénescente.

6. Méthode pour la réversion in vitro d'une cellule sénescente, comprenant l'application d'un inhibiteur de PDK1 à une cellule sénescente *in vitro,* dans laquelle l'inhibiteur de PDK1 est un inhibiteur de l'expression d'un gène de PDK1 ou un inhibiteur de l'activité d'une protéine de PDK1.

7. Méthode selon la revendication 6, dans laquelle l'inhibiteur de l'expression du gène de PDK1 est choisi dans le groupe constitué par des oligonucléotides antisens, des petits ARN interférents (ARNsi), des petits ARN en épingle à cheveux (ARNsh), et des ARNmi (microARN) se liant de manière complémentaire à l'ARNm d'un gène pour promouvoir l'expression de PDK1 ou un gène de PDK1.

8. Méthode selon la revendication 6, dans laquelle l'inhibiteur de l'activité de la protéine de PDK1 est choisi dans le groupe constitué par un anticorps, un fragment de celui-ci se liant à un antigène, un composé, un peptide, un peptidomimétique et un aptamère se liant spécifiquement à la protéine de PDK1.

9. Méthode selon la revendication 6, dans laquelle la cellule sénescente est un fibroblaste sénescent ou une cellule endothéliale vasculaire sénescente.

FIGURE 1

FIGURE 2

## Senescence to Quiescence Control: Inhibition (2000 Models)

FIGURE 3

FIGURE 4

FIGURE 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS,* 1001409-50-2